# EUROPÄISCHE PATENTANMELDUNG

(11) **EP 2 561 818 A1**
(43) Veröffentlichungstag der Anmeldung: **27.02.2013**
(21) Anmeldenummer: 12181219.2
(22) Anmeldetag: 21.08.2012
(51) Int. Cl.: A61B 18/04

(54) **System zur Dampfbehandlung von Blutgefäßen**

(30) Priorität: 22.08.2011 DE 202011104843 U
(71) Anmelder: Netzer, Florian, 80803 München (DE)
(72) Erfinder: Netzer, Florian, 80803 München (DE)
(74) Vertreter: Maiwald Patentanwalts GmbH

(57) **Zusammenfassung**

Die Erfindung betrifft ein System zur Bereitstellung von Dampf in Blutgefäßen, wobei ein erfindungsgemäßer Adapter die Verwendung von gängigen Kanülen, Infusionssystemen oder Venenverweilkathetern durch die Kopplung eines SVS-Systems mit einem Luer-Lock verbindet. Die Bereitstellung eines Luer-Konus ermöglicht dem Adapter, insbesondere kurze und/oder sehr gekrümmte Venen mittels Dampf zu behandeln.

## Beschreibung

### Gebiet der Erfindung

Die Erfindung betrifft die Venenbehandlung mit Hochdruckdampf. Insbesondere betrifft die Erfindung ein System zur Dampfbehandlung von Blutgefäßen.

### Technologischer Hintergrund

Unter dem Begriff steam veine sclerosis (SVS) ist eine Technik bekannt, die zur Behandlung insbesondere zur Obliteration von Venen mit heißem Dampf verwendet wird. Diese thermische Methode stellt eine Alternative zur Methode mit Radiofrequenz oder Lasern oder der Mikroschaumskleorsierung oder Sklerosierung (Verödung) mit flüssigen Substanzen zur Behandlung von erkrankten Venen dar.

Bei der SVS-Methode wird Wasser mit Druck in einen Schlauch eingespritzt, der im vorderen Teil des Schlauchs durch beispielsweise eine elektrische Vorrichtung erhitzt wird. Dadurch wird ermöglicht, dass erhitztes Wasser in Dampfform pulsartig, mit über 100 °C austreten kann. Insbesondere tritt der Dampf am Ende des Schlauchs an einem Handstück aus. Dazu wird ein Katheter mit dem Handstück verbunden, so dass der im Handstück erzeugte Dampf durch den Katheter an die entsprechend entlegene Stelle in der Vene geleitet werden kann. Der an dem Ende des Katheters austretende Dampf kondensiert wieder zu Wasser und die zurückbleibende Hitze wird sofort von der Blutgefäßwand, beispielsweise der Venenwand, absorbiert. Dabei wird SVS als besonders sicher betrachtet, da das Überhitzungslimit aufgrund des schnellen Hitzetransfers sehr hoch liegt.

Um das SVS-Verfahren vorzubereiten, wird die zu behandelnde Vene punktiert und über eine sogenannte Schleuse wird ein langer, dünner SVS-Katheter aus flexiblem Kunststoff eingeführt. Dieser Katheter hat eine Länge in der Größenordnung von etwa 60 bis 80 cm.

Anschließend wird der Schlauch unter der Abgabe von kurzen Stößen von sterilem Wasserdampf, der bei Austritt in der Vene circa 120 °C hat, langsam zurückgezogen. Typischerweise wird der SVS-Katheter dazu auf das Schraubgewinde des Handstücks aufgeschraubt. Dabei ist beim Einsatz von Kathetern zwingend die Einführung dieses Katheters über eine Schleuse verlangt. Dabei ist die Kombination aus SVS mit dem vorgesehenen Katheter lediglich zur Behandlung von Stammvenen oder anderen Venen geeignet, die vom Durchmesser und der Länge an gerader Strecke, die nötig ist um eine solche Schleuse einführen zu können wenigstens 5 cm betragen.

Bisher sind Besenreiser und andere kapillarartige und kapillare Gefäße nur der Verödung, dem Laser oder elektrischen Verfahren zugänglich. Nach einer Verödung jedoch, zum Beispiel mit flüssigem Wirkstoff oder als Mikroschaumsklerosierung, wobei das flüssige Agens mit Gas vermischt zu einem feinporigen Schaum gemischt wurde, müssen üblicherweise Kompressionsverbände und/oder Kompressionsstrümpfe der Klasse II getragen werden und es kann infolge der Behandlung eine ästhetisch beeinträchtigende Hyperpigmentierung auftreten. Bei der Laserbehandlung muss zwar keine Kompression angewendet werden, aber es kann zu einer Hyper- oder Depigmentierung kommen. Bei den in multipler Form hochfrequenter Wechselstromanwendung, auch Radiofrequenzanwendung wird das zu behandelnde Gefäß mit einer Nadel angestochen und dann durch Strom- und Hitzewirkung zerstört. Dazu sind sehr viele Einstiche und Sitzungen notwendig und die Ergebnisse der Behandlungen gelten allgemein als unsicher.

Viele übliche Systeme zur Zerstörung von Gewebe, z.B. epifaszialen Venen, beruhen in letzter Konsequenz auf dem Effekt thermischer Energie: Laser, Heißdampf, hochfrequente Ströme, etc. Die Systeme funktionieren unterschiedlich gut bei verschiedenen Venen, sind aber stets mit hohen Investitionskosten und erheblichem Behandlungsaufwand für Arzt und Patienten verbunden.

### Zusammenfassung der Erfindung

Es kann als eine Aufgabe der Erfindung angesehen werden, eine verbesserte Behandlung von Blutgefäßen mit Dampf zu ermöglichen.

Die Aufgabe der Erfindung wird mittels des Gegenstandes des unabhängigen Anspruchs gelöst. Weitere Vorteile und Weiterbildungen sind in den Unteransprüchen angegeben.

Gemäß einem Ausführungsbeispiel der Erfindung ist ein System zur Behandlung von Blutgefäßen mit Dampf angegeben. Das System weist eine Leitung mit einem Handstück zur Bereitstellung von Dampf in dem Blutgefäß auf. Weiterhin weist das System einen Adapter auf. Das Handstück weist ein vorderes Ende mit einem Verbindungselement auf und das Verbindungselement weist eine erste Öffnung auf. Weiterhin weist das Verbindungselement eine erste Befestigungsvorrichtung zur Anbringung von zum Beispiel einem Katheter auf. Weiterhin weist der Adapter eine zweite Befestigungsvorrichtung auf, welche zur Befestigung des Adapters an der ersten Befestigungsvorrichtung des Verbindungselements ausgeführt ist. Weiterhin weist der Adapter eine dritte Befestigungsvorrichtung zur Befestigung eines Gegenstandes an dem Adapter auf, wobei die dritte Befestigungsvorrichtung als Luer-Konus ausgeführt ist.

Dabei kann, falls gewünscht, dieses Ausführungsbeispiel in einer Erweiterung auch einen Dampfgenerator aufweisen. Darunter ist eine Einheit zu verstehen, die Dampf, insbesondere Wasserdampf, erzeugen kann. Daher ist das erfindungsgemäße System in diesem Fall als ein System zur Dampfgenerierung und Dampfbehandlung von Blutgefäßen zu sehen, welches auch die hierin beschriebenen Vorteile mit sich bringt. Der Dampf kann aber auch ein Gemisch aus Wasserdampf und Zusätzen wie Medikamenten oder Anderem sein.

Dabei ist im Kontext der vorliegenden Erfindung der Begriff "Befestigungsvorrichtung" als eine Vorrichtung zu betrachten, die zwei mechanische Gegenstände miteinander in Verbindung bringt und sie fixiert. Beispielsweise ist ein Form- und/oder ein Kraftschluss möglich. Beispielsweise kann eine Befestigungsvorrichtung ein Innengewinde, aber auch ein Außengewinde sein. Auch eine Mutter, wie zum Beispiel eine Überwurfmutter kann als eine Befestigungsvorrichtung im Kontext der vorliegenden Erfindung bezeichnet werden. Aber auch andere Vorrichtungen, die zur Befestigung dienen, können unter dem Begriff Befestigungsvorrichtung im Kontext der Erfindung verstanden werden.

Es wird daher auch ein System gemäß dem unabhängigen Anspruch bereitgestellt, welches den dort angegebenen Adapter mittels einer Überwurfmutter an dem Verbindungselement befestigt. Die Überwurfmutter kann also Teil des erfindungsgemäßen Systems sein. Die erste Befestigungsvorrichtung ist in diesem Fall als Gewinde ausgeführt, auf welches eine Überwurfmutter aufgeschraubt werden kann. Zuvor kann der Adapter auf oder über das Verbindungselement des Handstücks platziert werden. Beim Aufschrauben der Überwurfmutter wird dann der Adapter, der den Luer-Konus bereitstellt mit dem Handstück dampfdicht verbunden. Dazu kann der Adapter einen entsprechenden Bereich aufweisen, an den die Überwurfmutter beim Aufschrauben anschlägt und so den Adapter in Richtung des Handstücks drückt und fixiert.

Dabei wird im Kontext der Erfindung unter Luer-Lock und Luer-Konus ein genormtes Verbindungssystem für Schlauchsysteme im klinischen Bereich verstanden. Es können daher im Kontext der vorliegenden Erfindung Dichtungen durch kegelförmige Konstruktionen der Verbindungsteile, der sogenannten Luer-Konen, erreicht werden. Dabei wird der Innenkegel der einen Verbindungsseite auch als weiblich und der Außenkegel der Gegenseite als männlich bezeichnet. Falls gewünscht, kann der Konus zur Sicherung bzw. zur Verriegelung der Verbindung gegen versehentliches Lösen durch ein Gewinde mit einer Überwurfmutter erweitert werden. Beispielsweise kann diese Verbindung mit einer halben Drehung öffnen und schließen.

Mit anderen Worten, wird gemäß der Erfindung ein Kit aus einem SVS-System und einem erfindungsgemäßen Adapter bereitgestellt. Der erfindungsgemäße Adapter ermöglicht es, anstelle des langen und sehr teuren SVS-Katheters, der darüber hinaus immer die Einführung über die Schleuse verlangt, zum Beispiel eine Venenverweilkühlkanüle unterschiedlicher handelsüblicher Kaliber in eine zu behandelnde Vene einzuführen, und diese ebenfalls mit dem SVS-System zu behandeln. Neben diesem Vorteil ermöglicht die vorliegende Erfindung ebenso eine Behandlung von kurzen und/oder sehr gekrümmten Venen.

Insbesondere können mit diesen kurzen Venenverweilkathetern erstmals auch "Crossenrezidive" minimalinvasiv mit Heißdampf behandelt werden, die bisher ganz überwiegend oder ausschließlich nur der konventionellen "offenen Chirurgie", also mit Schnitt und Unterbindung und Venenentfernung behandelt werden konnten. Als "Crosse" bezeichnet der Venenarzt/chirurg den Übergang der oberflächlichen Stammvenen (epifaszialen Stammvenen: Vena saphena magna und parva oder Große und Kleine Rosenvene) in die inneren (tiefen) Leitvenen (Vena femoralis und Vena poplitea). Als Rezidiv bezeichnet man allgemein das erneute Auftreten einer bereits ausgeheilten Erkrankung, hier: den Zustand neuerlicher kranker (rückläufiger) Venen im Bereich dieser Übergänge nach operativer Sanierung derselben unter Entfernung der rückläufigen Venen. Diese "Rezidiv"-Venen können entweder Relikte einer vorausgegangenen Operation (also belassene Venen), oder aber auch echte Neubildungen infolge der "Angioneogenese" sein. Gerade Gefäße der letzteren Klasse zeichnen sich durch besonders komplizierte gekrümmte Verläufe auf und sind auch in der Regel deutlich dünnwandiger als bereits embryonal (vollschichtig aufgebaute) Venen und damit auch chirurgisch nur sehr schwer zu behandeln, weil sie bei Manipulation schnell einreißen und bluten. Hier ist ein Verfahren, bei welchem die Venen unter Ultraschallkontrolle punktiert und durch thermische Energie verschlossen werden kann aus genannten Gründen sehr günstig, es gibt dabei praktisch keine Blutungen. Außerdem liegen die o.g. "Crossen" (Leiste und meist Kniekehle) immer in Gebieten mit sehr wichtigen Lymphgefäßen und Lymphknoten, welche bei jeder offenen Operationstechnik unvermeidlich geschädigt werden. Auch diesen Nachteil vermeidet das hier vorgestellte Verfahren mittels der angegebenen Erfindung.

Dies ist mit dem im Stand der Technik bekannten System in Kombination mit langen Kathetern nicht möglich. Die vorliegende Erfindung ermöglicht es insbesondere, Venen, die sich bei Rezidiven an den Kreuzungen zwischen dem oberflächlichen und dem tiefen Venensystem an den Crossen bilden, mit Dampf zu behandeln. Ebenso ermöglicht die vorliegende Erfindung die bisher unzugänglichen Perforansvenen, also Venen, welche Kurzschlüsse zwischen dem oberflächlichen, epifaszialen und dem tiefen, subfaszialen System herstellen, mit Dampf zu behandeln.

In diesem und in jedem anderen Ausführungsbeispiel der Erfindung kann ein Schlauch oder Ähnliches und einen Dreiwegehahn aufweisen. Damit lassen sich zum Beispiel über eine sehr feine Kanüle auch so genannte Besenreiser und andere kapillarartige und kapillare Gefäße behandeln, die bisher nur der Verödung, dem Laser oder elektrischen Verfahren zugänglich waren. Dies ist ein weiterer, praxisrelevanter Vorteil der vorliegenden Erfindung, da damit die zuvor genannten Nachteile des jeweiligen Standes der Technik vermieden werden. Diese Nachteile weist das hier vorgestellte Ausführungsbeispiel der Erfindung allesamt nicht auf. Die Erfindung ermöglicht auch mit einem einzigen Gerät (SVS) alle zu behandelnden Venen zu therapieren: von Stammvenen bis zum kleinen Besenreiser, wozu andernfalls mehrere Geräte und meist viele Sitzungen nötig sind. Das kann sehr hohe Investitionskosten bedeuten. Mit dem hier beschriebenen Ausführungsbeispiel der Erfindung kann in einer einzigen Sitzung der größte Teil der Venen erfolgreich behandelt werden.

Dabei kann in diesem und in jedem anderen Ausführungsbeispiel anstelle des Schlauchs ein Gegenstand verwendet werden, der eine geführte Zuleitung eines Mediums aus dem Adapter in das zu behandelnde Blutgefäß erlaubt.

Die Verlängerung durch einen zwischen 5 und 20 cm langen Infusionsschlauch, z.B. Teil einer "Heidelberger Verlängerung" dient dabei einerseits dazu dem Dampf mit einer deutlich geringeren Temperatur an den sehr viel kleineren Gefäßen anzuwenden und zum anderen kommt es dadurch zu einer gewissen Druckminderung, die verhindern soll, dass die kleinen Gefäße platzen. Die gewünschte Temperatur kann dabei durch unterschiedliche Schlauchlängen realisiert werden. Der noch dazwischengeschaltete Dreiwegehahn kann dabei an der einen - unbesetzten - Seite mittels des Hahns gerade soweit geöffnet werden, wie es der Behandlor wünscht und somit kann eine weitere Druckreduktion erreicht werden. Zur Behandlung wird also z.B, zuerst der Dreiwegehahn auf den oben als Erfindung beschriebenen Adapter gesetzt, wobei der Luer-Ansatz des Dreiwegehahns dazu dient. Das zweite Anschlussstück des Dreiwegehahns bleibt unbesetzt, um es ggf. zur oben beschriebenen Druckminderung zu nutzen, an das dritte Anschlussstück wird schließlich der Infusionsschlauch aufgesetzt, den der Behandler entweder in verschiedenen Längen benutzen kann oder den er sich selbst entsprechend zurechtschneidet. An das freie Ende des Schlauches wird dann eine entsprechend sehr feine Injektionsnadel befestigt, z.B. eine "Insulinnadel". Nun hat der Behandler eine sehr flexible Behandlungseinheit, die er deshalb feinmotorisch genau durch Punktion der sehr feinen Gefäße mit der dünnen Nadel benutzen kann. Daher kann ein flexibler Schlauch weitere Vorteile mit sich bringen.

Eine Kombination aus der erfindungsgemäßen Leitung und dem erfindungsgemäßen Adapter ermöglicht es, preisgünstige Verweilkanülen zu verwenden, was die durchschnittlichen Kosten einer solchen Behandlung reduziert. Es findet dadurch also eine erhebliche Erweiterung der Indikationen des SVS-Geräts und der gesamten Heißdampfmethode statt. Mit anderen Worten, ermöglicht die Erfindung das vorteilhafte SVS-System bzw. die vorteilhafte SVS-Methode in ganz neuen und anderen, bisher nicht bekannten medizinischen Bereichen bzw. Bereichen von Blutgefäßen, anzuwenden.

Gemäß einem weiteren Ausführungsbeispiel der Erfindung ist der Adapter aus Edelstahl gefertigt, insbesondere ist der Adapter aus Chirurgenstahl gefertigt. Dabei können unterschiedliche Legierungen von beispielsweise Chrom, Molybdän, und, falls gewünscht, zusätzlichen Kohlenstoffanteilen verwendet werden.

Gemäß einem weiteren Ausführungsbeispiel der Erfindung ist die erste Befestigungsvorrichtung als ein Außengewinde ausgeführt und die zweite Befestigungsvorrichtung ist als entsprechendes Innengewinde ausgeführt. Mit anderen Worten, kann damit der Adapter auf das Verbindungselement des Handstücks der Leitung aufgeschraubt werden. Dabei können unterschiedliche Gewindedurchmesser und Gewindesteigungen verwendet werden.

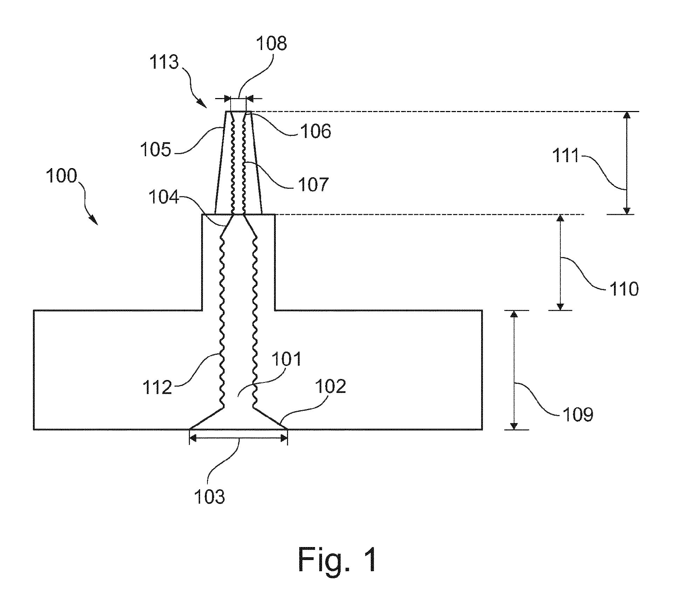

Gemäß einem Ausführungsbeispiel der Erfindung weist der Adapter zwei unterschiedliche Innengewinde auf, die beide jeweils unterschiedliche Durchmesser aufweisen. Mit diesem Ausführungsbeispiel kann gewährleistet werden, dass der Adapter einerseits auf das Gewinde des Verbindungselements des Handstücks aufgebracht werden kann, beispielsweise aufgeschraubt werden kann, und dass gleichzeitig eine Kompatibilität mit einem anderen Gegenstand mit einem anderen Gewindedurchmesser bereitgestellt wird. Dabei kann sich das zweite Innengewinde des Adapters beispielsweise in einem kegelförmigen Abschnitt des Adapters befinden, der als Luer-Konus dient. Dies kann beispielsweise der Fig. 1 entnommen werden.

Gemäß einem weiteren Ausführungsbeispiel der Erfindung weist das System eine Kanüle zur Einführung in ein Blutgefäß auf. Dabei kann dies beispielsweise eine Verweilkanüle sein.

Mittels des erfindungsgemäßen Adapters kann daher ein SVS-Gerät an eine preisgünstige Kanüle mechanisch angebunden werden, so dass durch diese Kanüle Dampfapplikation erfolgen kann. Mittels eines Luer-Konus des Adapters kann eine Kanüle verwendet werden, die zusammen mit dem Luer-Konus ein Führloch bildet.

Gemäß einem weiteren Ausführungsbeispiel der Erfindung weist die Kanüle einen konusförmigen Bereich auf und der konusförmige Bereich der Kanüle ist zur Befestigung der Kanüle an dem Luer-Konus des Adapters ausgeführt.

Aufgrund der Ausführung der dritten Befestigungsvorrichtung als Luer-Konus können gängige Kanülen, Infusionssysteme, Venenverweilkatheter und auch andere Geräte, die ein entsprechendes Gegenstück zum Luer-Konus aufweisen, an dem Adapter angebracht werden. Er erweitert sich, dadurch das Spektrum der SVS-Applikationen.

Gemäß einem weiteren Ausführungsbeispiel der Erfindung ist der Luer-Konus des Adapters als männliches Verbindungsteil eines Luer-Locks ausgeführt, und die Kanüle ist als weibliches Verbindungsteil des Luer-Locks ausgeführt.

Gemäß einem weiteren Ausführungsbeispiel der Erfindung sind die erste Befestigungsvorrichtung des Verbindungselements und die zweite Befestigungsvorrichtung des Adapters in Kombination derart zur gegenseitigen Befestigung ausgeführt, dass eine wasserdampfdichte Verbindung zwischen der ersten Öffnung der Leitung und dem Adapter entsteht.

Der Adapter ist damit nicht nur in der Lage, eine Kanüle über den Luer-Konus wasserdampfdicht an sich zu befestigen, sondern stellt auch eine wasserdampfdichte Verbindung zwischen dem Verbindungselement des Handstücks der Leitung und der aufzubringenden Kanüle bereit.

Als ein weiteres Ausführungsbeispiel der Erfindung weist das System weiterhin ein Rückschlagventil zur Verhinderung eines Eindringens von Flüssigkeit durch den Adapter in die Leitung auf, wobei das Rückschlagventil zwischen der Kanüle und dem Adapter angeordnet ist.

Das Rückschlagventil kann beispielsweise als steriler Einmalartikel ausgeführt sein. Solch ein Rückschlagventil wird üblicherweise verwendet, um Infusionen an einem Zurücklaufen zu hindern. Dadurch kann verhindert werden, dass Blut des Patienten in das Handstück rückgepresst wird, was sonst durch die Druckwelle des Dampfstoßes der Fall sein könnte. Dadurch kann auch verhindert werden, dass fragile Fremdgewebspartikel in einen Patienten eingebracht werden. Dadurch wird eine hygienisch einwandfreie SVS Methode mit dem oben genannten breiten und neuen Spektrum an Applikationen bereitgestellt.

Gemäß einem weiteren Ausführungsbeispiel der Erfindung weist der Adapter einen ersten kreisrunden, zylindrischen Abschnitt mit einem ersten Durchmesser auf und der Adapter weist einen zweiten kreisrunden, zylindrischen Abschnitt mit einem zweiten Durchmesser auf. Der Luer-Konus weist einen kreisrunden, kegelförmigen Abschnitt auf. Der erste Durchmesser beträgt zwischen 17 mm und 20 mm, insbesondere zwischen 18 mm und 19 mm, und weiter insbesondere 18,08 mm. Der zweite Durchmesser beträgt zwischen 5 mm und 8 mm, insbesondere zwischen 6 mm und 7 mm, und weiter insbesondere 6,08 mm. Der erste Abschnitt weist eine Höhe zwischen 2 mm und 8 mm auf, insbesondere weist der erste Abschnitt eine Höhe zwischen 5 mm und 6 mm, und weiter insbesondere weist der erste Abschnitt eine Höhe von 5,33 mm auf. Weiterhin weist der zweite Abschnitt eine Höhe zwischen 6 mm und 11 mm auf, insbesondere weist der zweite Abschnitt eine Höhe zwischen 8 mm und 9 mm, und weiter insbesondere weist der zweite Abschnitt eine Höhe von 8,91 mm auf.

Gemäß einem weiteren Ausführungsbeispiel der Erfindung ist das System zur Dampfgenerierung, zum Beispiel zur Wasserdampfgenerierung, und zur Bereitstellung von Dampf, zum Beispiel Wasserdampf, ausgeführt und weist einen Dampfgenerator, zum Beispiel einen Wasserdampfgenerator auf.

Gemäß einem weiteren Ausführungsbeispiel der Erfindung ist das System zur Bereitstellung von Wasserdampf an einer Austrittsöffnung einer Venenverweilkanüle ausgeführt ist. Das System weist eine Venenverweilkanüle mit einer Austrittsöffnung auf, wobei die Venenverweilkanüle mit dem Adapter in wasserdampfdichter Verbindung steht. Der Wasserdampfgenerator steht zur Bereitstellung von Wasserdampf an der Austrittsöffnung der Venenverweilkanüle mit der Leitung in wasserdampfdichter Verbindung.

Gemäß einem weiteren Ausführungsbeispiel der Erfindung ist das System ein System zur gezielten Gefäß- und/oder Gewebezerstörung mittels Heißperfusion. Das System weist eine Pumpvorrichtung und eine Erhitzungsvorrichtung auf. Dabei ist ein Medium mittels der Pumpvorrichtung über einen Schlauch der Erhitzungsvorrichtung zuführbar, wobei das Medium nach Durchgang durch die Erhitzungsvorrichtung dem Handstück zuführbar ist.

Zum Beispiel kann die Pumpvorrichtung eine Rollenpumpe oder eine Infusionspumpe sein. Die Erhitzungsvorrichtung kann zum Beispiel als Durchlauferhitzer ausgeführt sein. Aber auch andere Ausführungsformen sind möglich. Die Pumpvorrichtung leitet das flüssige Medium über einen dafür vorgesehenen Schlauch in die Erhitzungsvorrichtung. Falls gewünscht kann diese mit variabler, steuerbarer Temperatureinstellung versehen sein. Damit kann die gewünschte Temperaturhöhung des Mediums erreicht werden. Anschließend kann das Medium zum Beispiel über einen gut isolierten Schlauch, zum Beispiel aus Einwegmaterial, zum Patienten geführt werden. Beispielsweise kann das durch den Schub der genannten Pumpvorrichtung, zum Beispiel eine Rollenpumpe, erfolgen.

Der Arzt führt eine Venenverweilkanüle in einem dem Venendurchmesser angepassten Kaliber in die zu zerstörende Vene ein und leitet so das erhitzte Medium in die Vene, wobei zuvor um die Vene herum eine isolierende Schicht aus physiologischer Kochsalzlösung mit oder ohne Zusatz von Lokalanästhetikum gespritzt wurde, um eine Verletzung von Begleitstrukturen zu verhindern. Es kann auch ein anderer Katheter zum Einsatz kommen, der ca. 100 cm lang ist und in die Vene eingeführt und dann - unter ständiger Perfusion mit der heißen Lösung - zurückgezogen wird.

Damit ist eine sehr viel einfachere Behandlung möglich, da man z.B. Venen durch eine Heißperfusion mit z.B. physiologischer Kochsalzlösung oder Aqua destillata behandeln kann. Dabei ist das oben genannte Medium hier als Kochsalzlösung oder Aqua destillata zu verstehen. Aber auch andere Medien sind mit dem erfindungsgemäßen System verwendbar. Die Perfusion mit Aqua destillata hat den zusätzlichen Effekt, dass lebende Zellen das hypotone Aqua destilata solange in sich aufnehmen, bis sie alleine daran zugrunde gehen können.

Mit anderen Worten ist in diesem Ausführungsbeispiel die Pumpvorrichtung mit der Erhitzungsvorrichtung mittels eines ersten Verbindungselements, zum Beispiel einem Schlauch, verbunden. Weiterhin ist die Erhitzungsvorrichtung mit dem erfindungsgemäßen Handstück mittels eines zweiten Verbindungsstück, zum Beispiel einem Schlauch, verbunden. Falls gewünscht, können diese Verbindungen auch wasserdampfdicht ausgeführt sein. Weiterhin kann das System einen Behälter mit einer Kochsalzlösung oder Aqua destilata aufweisen. Eine entsprechende Zufuhr zu der Erhitzungsvorrichtung ist ebenso bereitgestellt.

Dieses Ausführungsbeispiel lässt sich, falls nicht explizit anders hierin genannt, mit den übrigen Ausführungsbeispielen der vorliegenden Erfindung kombinieren.

Der Adapter dieses und jedes anderen Ausführungsbeispiels erlaubt ein verlustfreies Überführen von Dampf von der Leitung durch den Adapter bis in den darauf aufgebrachten Gegenstand wie zum Beispiel eine Verweilkanüle für eine Vene.

Die Erfindung wird im Folgenden unter Hinweis auf die beigefügten Figuren anhand schematischer Darstellungen bevorzugter Ausführungsbeispiele noch einmal näher erläutert. Hieraus ergeben sich auch weitere Einzelheiten und Vorteile der Erfindung.

### Kurze Beschreibung der Figuren

Die Darstellungen in den Figuren sind schematisch und nicht maßstäblich. In den Figurenbeschreibungen werden für die gleichen oder ähnlichen Elemente die gleichen Bezugsziffern verwendet.

Ergänzend ist darauf hinzuweisen, dass "umfassend" keine anderen Elemente oder Schritte ausschließt und "ein" oder "eine" keine Vielzahl ausschließt. Ferner sei darauf hingewiesen, dass Merkmale oder Schritte, die mit Verweis auf eines der obigen Ausführungsbeispiele beschrieben worden sind, auch in Kombination mit anderen Merkmalen oder Schritten anderer oben beschriebener Ausführungsbeispiele verwendet werden können. Bezugszeichen in den Ansprüchen sind nicht als Einschränkung anzusehen.
Fig. 1 zeigt einen schematischen, zweidimensionalen Querschnitt eines Adapters gemäß einem Ausführungsbeispiel der Erfindung.
Fig. 2 zeigt eine schematische, zweidimensionale Draufsicht auf einen Adapter gemäß einem Ausführungsbeispiel der Erfindung.
Fig. 3 zeigt eine schematische, zweidimensionale Darstellung eines Handstücks eines SVS-Systems gemäß einem Ausführungsbeispiel der Erfindung.
Fig. 4 und 5 zeigen schematische, zweidimensionale Darstellungen eines Adapters gemäß einem Ausführungsbeispiel der Erfindung.
Fig. 6 zeigt eine schematische, zweidimensionale Darstellung einer Leitung gemäß einem Ausführungsbeispiel der Erfindung.

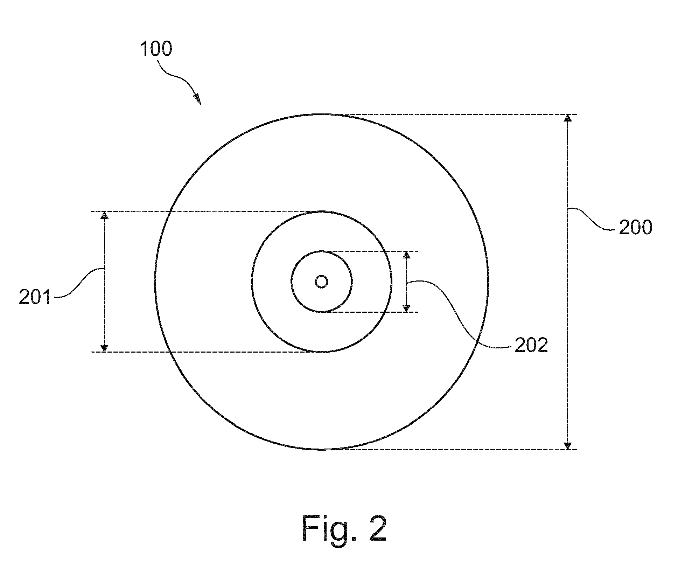

Fig. 1 zeigt einen Querschnitt eines Adapters 100, der als zweite Befestigungsvorrichtung das Innengewinde 101 aufweist und der aus Chirurgenstahl gefertigt ist. Es wird weiterhin gezeigt, dass das Innengewinde 101 eine Fase 102 aufweist. Dadurch erhöht sich der Durchmesser der Öffnung 103 des Adapters, was durch den Abstand mittels des gezeigten Pfeils verdeutlicht wird. Ebenso zeigt Fig. 1, dass am oberen Ende des Innengewindes 101 eine weitere Fase 104 angeordnet ist. Mit 105 ist ein kreisrunder, kegelförmiger Abschnitt des Adapters gezeigt, der die dritte Befestigungsvorrichtung 113 darstellt, welche als Luer-Konus ausgeführt ist. Die zweite Befestigungsvorrichtung 112 des Adapters 100 stellt das Innengewinde 101 dar. Ebenso ist der Fig. 1 zu entnehmen, dass der Adapter 100 ein zweites Innengewinde 107 aufweist, das einen anderen Durchmesser hat als das darunter befindliche erste Innengewinde 101. Ebenso ist eine dritte Faser 106 am Ende des zweiten Innengewindes 107 dargestellt. Es wird eine Öffnung 108 am oberen Ende des Adapters bereitgestellt. Dem rechten Bildrand der Fig. 1 ist zu entnehmen, dass der Adapter einen ersten kreisrunden, zylindrischen Abschnitt 109 und einen zweiten kreisrunden, zylindrischen Abschnitt 110 aufweist. Die beiden Abschnitte 109 und 110 weisen unterschiedliche Durchmesser auf Dies kann detailliert der nachfolgenden Fig. 2 entnommen werden. Durch die Ausgestaltung der dritten Befestigungsvorrichtung 113 als Luer-Konus können an ein SVS-Gerät anstatt eines Katheters gängige Kanülen, wie Verweilkanülen, oder auch Infusionssysteme an den Adapter angeschlossen werden. Damit können auch kurze und/oder sehr gekrümmte Venen nunmehr mit der Dampfmethode behandelt werden.

Das in Fig. 1 dargestellte System könnte zu einem System zur gezielten Gefäß- und/oder Gewebezerstörung mittels Heißperfusion gemäß einem weiteren Ausführungsbeispiel erweitert werden. Das System weist dann eine Pumpvorrichtung und eine Erhitzungsvorrichtung auf. Dabei ist ein Medium mittels der Pumpvorrichtung über einen Schlauch der Erhitzungsvorrichtung zuführbar, wobei das Medium nach Durchgang durch die Erhitzungsvorrichtung dem Handstück zuführbar ist.

Fig. 2 zeigt eine Draufsicht auf einen Adapter 100 aus Fig. 1. Hier ist zu erkennen, dass der erste Abschnitt 109 einen ersten Durchmesser 200 aufweist und dass der zweite Abschnitt 110 einen zweiten Durchmesser 201 aufweist. Ebenso ist für den als Luer-Konus ausgeführten kegelförmigen Bereich 105 ein Durchmesser 202 in Fig. 2 eingezeichnet.

Gemäß einem weiteren Ausführungsbeispiel sind die in Fig. 1 und 2 gezeigten Abmessungen wie folgt: die Höhe des ersten Abschnitts 109 beträgt 5,33 mm, die Höhe des zweiten Abschnitts 110 beträgt 8,91 mm, und die Höhe des dritten Abschnitts 111 beträgt 8,27 mm. Weiterhin beträgt der erste Durchmesser (200) 18,08 mm und der zweite Durchmesser 201 beträgt 6,08 mm. Für den Durchmesser 202 des Luer-Konus wird auf die dafür spezifizierte Norm verwiesen.

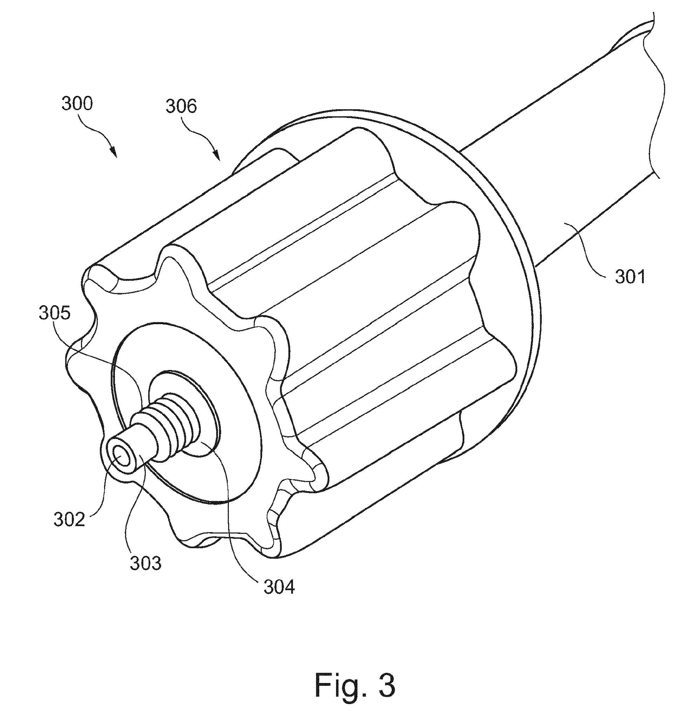

Fig. 3 zeigt eine Leitung 300 mit einem Handstück 306 zur Behandlung von Blutgefäßen mit Dampf. Dabei stellt die Leitung 301 Wasser dem Handstück 306 zur Verfügung, wo dieses bis zum Übergang in die Dampfphase erhitzt wird. Dabei ist das Verbindungselement 303 gezeigt, welches eine erste Öffnung 302 aufweist. Durch diese kann der erzeugte Dampf ausströmen und in den darauf aufzuschraubenden Adapter einströmen. Als erste Befestigungsvorrichtung 305 weist das in Fig. 3 gezeigte Ausführungsbeispiel ein Außengewinde auf. Der in Fig. 1 und 2 dargestellte Adapter kann auf das Handstück 306 der Fig. 3 aufgeschraubt werden, damit anschließend beispielsweise eine Kanüle auf den Luer-Konus des Adapters angebracht werden kann. Damit wird insgesamt ermöglicht, kurze und/oder sehr gekrümmte Venen oder auch die zuvor beschriebenen neuen Applikationen des SVS-Systems zu realisieren. Das Ausführungsbeispiel der Fig. 3 kann ein Schlauch oder Ähnliches und einen Dreiwegehahn aufweisen. Damit lassen sich zum Beispiel über eine sehr feine Kanüle auch so genannte Besenreiser und andere kapillarartige und kapillare Gefäße behandeln, die bisher nur der Verödung, dem Laser oder elektrischen Verfahren zugänglich waren. Dies ist ein weiterer, praxisrelevanter Vorteil der vorliegenden Erfindung

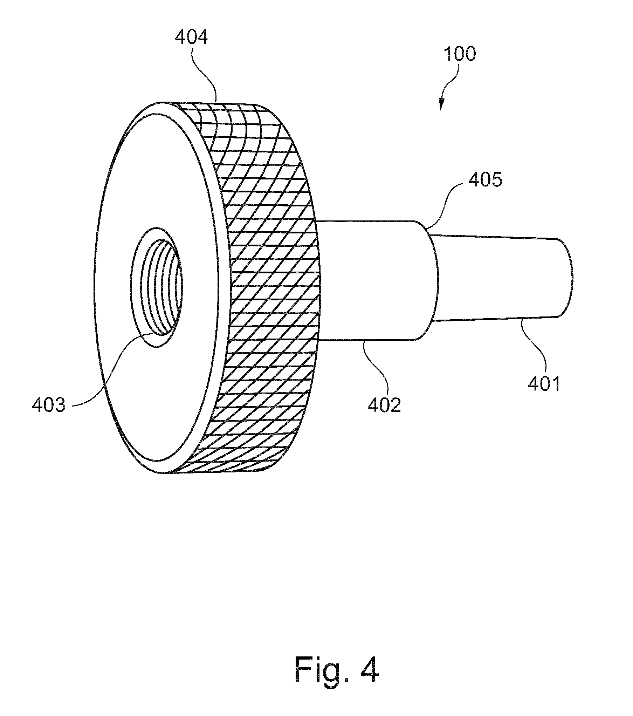

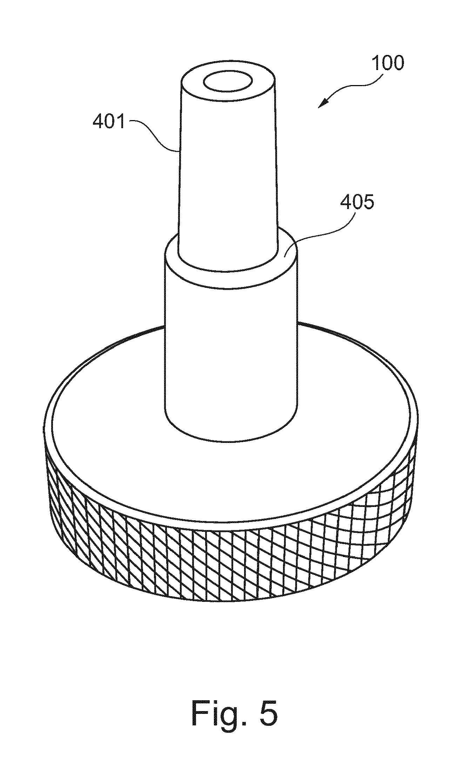

Die Fig. 4 und 5 zeigen beide unterschiedliche Ansichten eines Adapters 100, der ein Innengewinde 403 aufweist, mit welchem dieser an dem Handstück der Leitung 300 wasserdampfdicht angebracht werden kann. Dazu ist eine raue Oberfläche auf der gezeigten Fläche 404 generiert worden. Ebenso ist ein zylindrischer Bereich 402 gezeigt, von dem aus sich der Luer-Konus 401 erstreckt. Hierbei entsteht eine schlagkante 405. Fig. 5 zeigt den gleichen Adapter 100, wobei aus dieser Perspektive die konische Form des Luer-Konus 401 deutlich zu sehen ist. Auch ist die Anschlagkante 405 aus dieser Perspektive leicht zu erkennen.

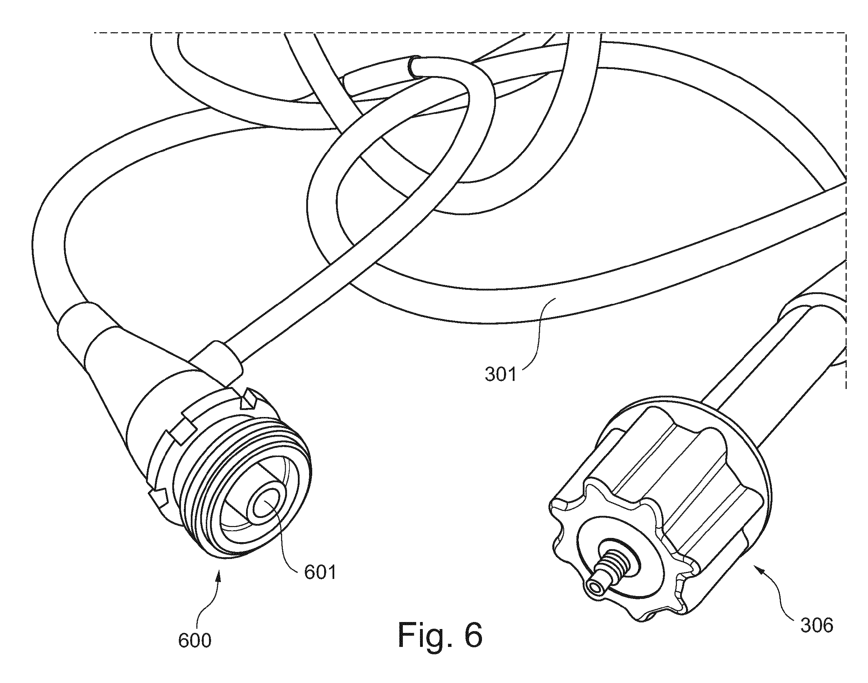

Fig. 6 zeigt eine Leitung 300 zur Bereitstellung von Dampf in einem Blutgefäß. Hier ist die Schlauchleitung 301 gezeigt, welche als Zuleitung für das Handstück 306 fungiert. Das Endstück 600 wird über das Schraubgewinde mit dem SVS-Generator der Firma CERMA® verbunden. Hierüber und über den rechteckig vom Endstück abzweigenden kurzen Schlauch wird der Schlauch und somit auch das Handstück aus dem Generator sowohl mit elektrischem Strom zur Erzeugung des Dampfs im Handstück, als auch mit sterilem Wasser und Pressluft zur Erzeugung der Dampfstöße versorgt und mit der Regeleinrichtung des Generators verbunden, damit der Operateur mittels Pedal die Dampfstöße auslösen kann.

In diesem wie auch jedem anderen Ausführungsbeispiel der Erfindung können ein Dampfgenerator und/oder eine entsprechender Tank, insbesondere ein Wassertank, als Merkmal enthalten sein. Aber auch eine Zuführung von Wasser und/oder anderen Substanzen, wie zum Beispiel zusätzlichen Medikamenten, mit entsprechenden Leitungen zu dem Dampfgenerator ist möglich.

## Patentansprüche

1. System zur Dampfbehandlung von Blutgefäßen, das System aufweisend:
eine Leitung (300) mit einem Handstück (306) zur Bereitstellung von Dampf in dem Blutgefäß,
einen Adapter (100),
wobei das Handstück ein vorderes Ende mit einem Verbindungselement (303) aufweist,
wobei das Verbindungselement eine erste Befestigungsvorrichtung (305) aufweist,
wobei der Adapter eine zweite Befestigungsvorrichtung (112) aufweist, welche zur Befestigung des Adapters an der Leitung an der ersten Befestigungsvorrichtung ausgeführt ist,
wobei der Adapter eine dritte Befestigungsvorrichtung (113) zur Befestigung eines Gegenstandes an dem Adapter aufweist, und
wobei die dritte Befestigungsvorrichtung als Luer-Konus ausgeführt ist.

2. System gemäß Anspruch 1,
wobei der Adapter aus einem Edelstahl besteht, der eine Werkstoffnummer (WNr.) aufweist, die ausgewählt ist aus der Gruppe bestehend aus WNr. 1.4003, WNr. 1.4006, WNr. 1.4016, WNr. 1.4021, WNr. 1.4104, WNr. 1.4301, WNr. 1.4305, WNr. 1.4306, WNr. 1.4307, WNr. 1.4310, WNr. 1.4316, WNr. 1.4401, WNr. 1.4404, WNr. 1.4440, WNr. 1.4435, WNr. 1.4452, WNr. 1.4462, WNr. 1.4541, WNr. 1.4571, WNr. 1.4581, WNr. 1.4841, und WNr. 1.7218.

3. System gemäß einem der Ansprüche 1 oder 2,
wobei die erste Befestigungsvorrichtung als ein Außengewinde ausgeführt ist, und
wobei die zweite Befestigungsvorrichtung als dem Außengewinde entsprechendes Innengewinde ausgeführt ist.

4. System gemäß einem der Ansprüche 1 bis 3,
wobei der Adapter zwei unterschiedliche Innengewinde aufweist, und
wobei die beiden Innengewinde unterschiedliche Durchmesser aufweisen.

5. System gemäß einem der Ansprüche 1 bis 4, das System weiterhin aufweisend,
eine Kanüle zur Einführung in ein Blutgefäß, und
wobei die Kanüle konisch ausgeführt ist, so dass sie zu dem Luer-Konus des Adapters als Gegenstück ausgeführt ist, wodurch ein Luer-Lock bereitgestellt wird.

6. System gemäß Anspruch 5
wobei die Kanüle einen konusförmigen Bereich aufweist, und
wobei der konusförmige Bereich der Kanüle zur Befestigung der Kanüle an dem Luer-Konus des Adapters ausgeführt ist.

7. System gemäß einem der Ansprüche 5 oder 6,
wobei der Luer-Konus des Adapters als männliches Verbindungsteil eines Luer-Locks ausgeführt ist, und
wobei die Kanüle als weibliches Verbindungsteil des Luer-Locks ausgeführt ist.

8. System gemäß einem der Ansprüche 1 bis 7,
wobei die erste Befestigungsvorrichtung der Leitung und die zweite Befestigungsvorrichtung des Adapters in Kombination derart zur gegenseitigen Befestigung ausgeführt sind, dass eine wasserdampfdichte Verbindung zwischen der ersten Öffnung der Leitung und dem Adapter entsteht.

9. System gemäß einem der Ansprüche 5 bis 8, das System weiterhin aufweisend:
ein Rückschlagventil zur Verhinderung eines Eindringens von Flüssigkeit durch den Adapter in die Leitung, und
wobei das Rückschlagventil zwischen der Kanüle und dem Adapter angeordnet ist.

10. System gemäß einem der Ansprüche 1 bis 9,
wobei der Adapter einen ersten kreisrunden, zylindrischen Abschnitt (109) mit einem ersten Durchmesser (201) aufweist,
wobei der Adapter einen zweiten kreisrunden, zylindrischen Abschnitt (110) mit einem zweiten Durchmesser (202) aufweist,
wobei der Luer-Konus einen kreisrunden, kegelförmigen Abschnitt aufweist,
wobei der erste Durchmesser zwischen 17 mm und 20 mm, insbesondere zwischen 18 mm und 19 mm beträgt,
wobei der zweite Durchmesser zwischen 5 mm und 8 mm, insbesondere zwischen 6 mm und 7 mm beträgt,
wobei der erste Abschnitt eine Höhe zwischen 2 mm und 8 mm, insbesondere zwischen 4 mm und 5 mm aufweist, und
wobei der zweite Abschnitt eine Höhe zwischen 6 mm und 11 mm, insbesondere zwischen 8 mm und 9 mm aufweist.

11. System gemäß einem der Ansprüche 1 bis 10,
wobei das System zur Dampfgenerierung, insbesondere zur Wasserdampfgenerierung, und zur Bereitstellung von Dampf in einem Blutgefäß ausgeführt ist, das System aufweisend:
einen Dampfgenerator, insbesondere einen Wasserdampfgenerator.

12. System gemäß Anspruch 11,
wobei das System zur Bereitstellung von Dampf, insbesondere Wasserdampf, an einer Austrittsöffnung einer Venenverweilkanüle ausgeführt ist; das System aufweisend:
eine Venenverweilkanüle mit einer Austrittsöffnung;
wobei die Venenverweilkanüle mit dem Adapter in dampfdichter Verbindung steht;
wobei der Dampfgenerator mit der Leitung in dampfdichter Verbindung zur Bereitstellung von Dampf an der Austrittsöffnung der Venenverweilkanüle steht.

13. System nach einem der Ansprüche 1 bis 12, weiterhin aufweisend
einen Schlauch, und
einen Dreiwegehahn.

14. System nach einem der Ansprüche 13,
wobei der Dreiwegehahn einen Luer-Konus aufweist.

15. System nach einem der Ansprüche 1 bis 14, wobei das System ein System zur gezielten Gefäß- und/oder Gewebezerstörung mittels Heißperfusion ist, das System weiterhin aufweisend
eine Pumpvorrichtung,
eine Erhitzungsvorrichtung,
wobei mittels der Pumpvorrichtung über einen Schlauch ein Medium der Erhitzungsvorrichtung zuführbar ist, und
wobei das Medium nach Durchgang durch die Erhitzungsvorrichtung dem Handstück zuführbar ist.
